Europäisches Patentamt

**European Patent Office**

**Office européen des brevets**

(11) Publication number: **0 228 036**
A2

(12) **EUROPEAN PATENT APPLICATION**

(21) Application number: **86117649.3**

(22) Date of filing: **18.12.86**

(51) Int. Cl.⁴: **C 12 N 15/00,** C 12 P 21/02,
C 12 N 5/00

(30) Priority: **18.12.85 US 810938**

(43) Date of publication of application: **08.07.87**
**Bulletin 87/28**

(84) Designated Contracting States: **AT BE CH DE ES FR GB
GR IT LI LU NL SE**

(71) Applicant: **Microgenesys, Inc., 400 Frontage Road, West
Haven New Haven Connecticut 06516 (US)**

(72) Inventor: **Cochran, Mark A., 415 Brooksvale Avenue,
Hamden Connecticut 06518 (US)**

(74) Representative: **Vossius & Partner,
Siebertstrasse 4 P.O. Box 86 07 67,
D-8000 München 86 (DE)**

(54) **Method for producing selected polypeptides in virally infected insect cells and polypeptides isolated therefrom.**

(57) A method for producing in a suitable insect host cell a selected polypeptide which comprises isolating a first DNA segment including a viral promoter from a virus capable of infecting said insect host cell, isolating from a suitable source a second DNA segment containing the sequence encoding said polypeptide, combining said first and second DNA segments to form a continuous strand of a third DNA segment including vector DNA in which said second DNA segment lies adjacent to and is in-frame with said promoter of said first DNA segment and contains transcription termination signals, forming a recombination vector by effecting recombination between said third segment and baculovirus genomic DNA, contacting said recombination vector so produced with said insect host cell under conditions such that said recombination vector segment becomes incorporated into said insect host cell to produce a treated insect host cell, and growing the host cell so treated and isolating the selected polypeptide from said host cell or culture fluids.

Further, a method for producing in a suitable insect host cell Hepatitis B virus surface antigen.

EP 86 11 7649.3
Our Ref.: W 117 EP
Case: 23546
Microgenesys, Inc.

*0228036*

## METHOD FOR PRODUCING SELECTED POLYPEPTIDES IN VIRALLY INFECTED INSECT CELLS AND POLYPEPTIDES ISOLATED THEREFROM

Throughout this application various publications are referenced by number within parentheses. Full citations for these publications may be found at the end of the specification immediately preceding the claims. The disclosures of these publications in their entireties are hereby incorporated by reference into this application in order to more fully describe the state of the art to which the invention pertains.

The potential utility of baculoviruses in pest management initially provided the impetus for their study; advances in the molecular biology of baculoviruses have sparked commercial interest. Information concerning the genomic organization and gene expression of baculoviruses is directly applicable to the genetic manipulation of the virus-host system to produce better pest control agents and to the use of these viruses as eukaryotic cloning vectors for the expression of foreign DNA. Furthermore, baculoviruses are proving to be useful tools in studies on the regulation of gene expression in invertebrate cells.

The family Baculoviridae consists of a single genus, Baculovirus, which is divided into 3 subgroups based on viral morphology.[1] Subgroup A viruses, the nuclear polyhedrosis viruses (NPV), produce virions which have

-2-

either a single nucleocapsid per envelope (SNPV), or one to many nucleocapsids per envelope (MNPV). Occlusion bodies (OB) form in the nucleus of NPV-infected cells, and many enveloped nucleocapsids (virions) are embedded in each OB. Subgroup B viruses, the granulosis viruses (GV), contain only one nucelocapsid per envelope and a single virion per OB. Subgroup C consists of singly enveloped nucleocapsids which are not occluded. Baculoviruses from subgroups A, B, and C have double-stranded, circular DNAs of about 100 to 150 kilobase pairs (kbp).

A major characteristic of baculoviruses from subgroups A and B is that crystalline occlusion bodies which contain occluded virus particles form in the nuclei of infected cells. The major protein of occlusion bodies, polyhedrin, is a polypeptide of molecular weight of about 30,000, which accounts for approximately 95% of the protein mass of occlusion bodies. A second form of the virus, a single enveloped nucleocapsid (extracellular nonoccluded virus, ENOV), is responsible for systemic spread in tissue culture. In the infected cell, most of the ENOV is synthesized prior to the virus occlusion process (86).

Baculovirus nomenclature is derived from the morphological subgroup of the virus and also from the insect of isolation. In this manner, a MNPV isolated from the alfalfa looper caterpillar _Autographa californica_ is designated AcMNPV. Selected baculoviruses with their designations are listed in Table 1.

The citations in Table 1 clearly indicate that the AcMNPV genomic variants have emerged as the model system for investigations on the molecular biology of

TABLE 1.    BACULOVIRUSES

| Virus Abbreviation | Host of Isolation | References |
|---|---|---|
| MNPV: | | |
| AcMNPV Genomic Variants – | | |
| AcMNPV-E | *Autographa californica* | 4, 4, 11, 20, 42, 45, 63, 79, 84 |
| AcMNPV-E2 | *Autographa californica* | 2, 5-8, 19, 28, 29, 31, 39, 41, 47, 50, 72, 81, 85 |
| AcMNPV-HR3 | *Autographa californica* | 5, 29, 40, 46, 57, 59, 64-66 |
| AcMNPV-L1 | *Autographa californica* | 3, 5, 9, 29, 51, 58 60-62, 67, 82 |
| GmMNPV | *Galleria mellonella* | 6 |
| RoMNPV | *Rachiplusia ou* | 8, 9 |
| SeMNPV-25 | *Spodoptera exempta* | 7 |
| TnMNPV | *Trichoplusia ni* | 6 |
| Other MNPV– | | |
| AgMNPV | *Anticarsa gemmitalis* | 85 |
| BmNP | *Bombyx mori* | 76 |
| BrMNPV | *Barathra brassicae* | 37 |
| CfMNPV | *Choristoneura fumiferana* | 25, 27, 30, 42, 73, 85 |

Table 1.
page 2

| Virus Abbreviation | Host of Isolation | References |
|---|---|---|
| HaMNPV | Heliothis armigera | 85 |
| MbMNPV | Mamestra brassicae | 31 |
| OpMNPV | Orgyia pseudotsugata | 9, 36, 38, 73, 78, 85 |
| PdMNPV | Porthetria dispar | 85 |
| PsMNPV | Pseudoletia seperata | 85 |
| SeMNPV | Spodoptera exigua | 85 |
| SfMNPV | Spodoptera frugiperda | 22-24, 34, 35 |
| SNPV: | | |
| AoSNPV | Adoxophyes orana | 37 |
| HzSNPV | Heliothis zea | 26, 85 |
| OpSNPV | Orgyia pseudotsugata | 36, 78 |
| PiSNPV | Pseudoplusia includens | 85 |
| TnSPNPV | Trichoplusia ni | 85 |
| Granulosis viruses: | | |
| HaGV | Heliothis armigera | 85 |
| PbGV | Pieris brassicae | 75 |
| PiGV | Plodia interpunctella | 85 |

Table 1.

page 3

| Virus Abbreviation | Host of Isolation | References |
|---|---|---|
| SfGV | *Spodoptera frugiperda* | 85 |
| TnGV | *Trichoplusia ni* | 85 |
| Persistent virus :<br>Hzl | *Heliothis zea* | 31 |

baculoviruses. The reason for its emergence is not because AcMNPV is more important than other baculoviruses; rather, it happens to be a system that is quite amenable to in vitro manipulations. Thus, the majority of the data presented herein was derived from analyses of the AcMNPV genomic variants.

The molecular characterization and physical mapping of other baculoviruses has lagged behind AcMNPV. Maruniak et al.[22] have mapped the DNAs of SfMNPV and its genomic variants using the restriction enzymes BamHI, BglIII, BstEII, EcoRI, HindIII, KpnI, and PstI. The genomic location of insertions and deletions in the variant DNAs was confined to four regions on the SfMNPV genome. These data corresponded with the studies of Knell and Summers,[23] in which they analyzed SfMNPV DNA obtained from four different laboratories, and with the HindIII and BamHI map derived by Loh and colleagues.[24] The physical map for SfMNPV presented by Maruniak et al.[22] was oriented with respect to the EcoRI fragment which contained sequences which were homologous to the AcMNPV polyhedrin gene.

In addition to AcMNPV and SfMNPV and their respective genomic variants, physical maps have also been derived for CfMNPV,[25] HzSNPV,[26] and OpMNPV.[27] These analyses are an important beginning to the genetic characterization of baculoviruses, and they will lead ultimately to elucidation of the general rules governing the biology of baculoviruses.

One direct benefit of physical mapping studies has been the establishment of restriction fragment libraries in various cloning vectors.[5,11,28] These reagents will facilitate the study of molecular biology of AcMNPV.

For example, they provide quantities of pure DNA from regions of the genome for the purposes of transcription and translation mapping and for the development of functional maps techniques such as marker rescue in the characterization of mutants prepared by *in vitro* mutagenesis.

The genome of AcMNPV and of most- other baculoviruses could encode as many as 100 proteins. About 40 viral induced proteins have been detected in infected cells (88), and more than 100 virus structural proteins have been resolved by 2-dimensional gel electrophoresis.[43,44]

The appearance of viral induced polypeptides appears to fall into at least four temporal classes:[45-50] Immediate early (2 h), early (2-6 h), late (8-12 h) and delayed late (12h and on). The early polypeptides are induced before DNA replication which begins at about 6 hours post-infection.[35] The late proteins, synthesized after the onset of viral DNA replication are mainly virion structural proteins, while the delayed late polypeptides are associated with the viral occlusion process (e.g., polyhedrin). A few of the early proteins are switched off during DNA replication while most of the others continue to be synthesized until late in the infection cycle. Each temporal class appears to require the proper expression of proteins from the preceding class.[49,51] In addition to temporal controls, control is also evident in the level of expression because the viral induced proteins were not synthesized in equimolar amounts in infected cells.

An endogenous RNA polymerase activity associated with either extracellular nonoccluded virus or occluded virus has not been reported. Since the NPV DNA is infectious, host RNA polymerase(s) must be responsible for transcribing viral RNA, at least early in infection. A number of animal nuclear DNA viruses, such as adenovirus, herpes simplex virus, and SV40, utilize the host RNA polymerase II which transcribes most of the viral mRNA and which is responsible for normal cellular mRNA synthesis.

Only one report examined the role of host RNA polymerase activities in NPV infected cells.[56] During AcMNPV infection of S. frugiperda cells, most of the AcMNPV-specific mRNA synthesis was resistant to alpha-amanitin, a potent inhibitor of RNA polymerase II. Thus, AcMNPV mRNA synthesis appears to be due to the activity of an RNA polymerase other than RNA polymerase II. In view of the atypical nature of baculovirus promoters which have been sequenced, the existence of a baculovirus-specific RNA polymerase involved with late-transcription is likely (87).

A number of distinct proteins have been synthesized _in vitro_ from poly(A)$^+$ RNA purified from late infected cells. The identity of a 30K dalton polypeptide known as polyhedrin was established by electrophoretic mobility and immunoprecipitation[41,50,54] and by peptide mapping.[40] The observation that _in vivo_ and _in vitro_ synthesized polyhedrins comigrate in gels confirms that this protein is not post-translationally modified to any great degree.[46,47] The identity of one of the major nucleocapsid proteins, 41K, has also been established.[50] These results support previous conclusions from recombinational studies that both proteins are virus encoded.[39]

A low-molecular weight methionine-deficient basic protein, designated the 10K protein, is produced in large quantities late in infection and appears to be expressed coordinately with polyhedrin in infected cells.[40,41,55] The function of the 10K protein is unknown. Since the majority of the 10K protein is non-structural and it is produced late in the infected cell at the same time as polyhedrin, it may be involved in the occlusion process. There is some evidence to suggest that the 10K protein is a minor structural component of the virus particle.[28]

There are several applications for constructing recombinant baculoviruses: 1) High efficiency eukaryotic expression vectors, as a means of producing selected polypeptides, 2) study of baculovirus gene function and organization, and 3) studying the regulation of gene expression in baculoviruses and/or invertebrates in general.

The most attractive application of the baculovirus vector system is for expressing large amounts of foreign gene product. The polyhedrin (Pn) gene is expressed more abundantly than any other gene in virus-infected eukaryotic cells. More than 15% of the protein mass found in a NPV infected larvae was polyhedrin,[78] and it was found by gel electrophoresis to represent 25% of the total protein mass in infected tissue culture cells.[41] Thus, these estimates represent the theoretical limits that may be anticipated in the expression of foreign genes from the natural polyhedrin promoter. This level could be exceded by genetic manipulation of sequences within the polyhedrin promoter.

In addition to the strong polyhedrin promoter sequences, several other features of the baculovirus genome make it an attractive agent for the cloning and expression of foreign DNA. These features include: 1) Baculoviruses do not represent a significant health hazard because they do not replicate in mammalian cells[79] or in vertebrate cells in general (96,97); 2) They exhibit a host range limited to certain invertebrates; 3) The baculovirus genome has been mapped for several restriction endonucleases and characterized with respect to its transcription activity; 4) Baculovirus gene products are subject to modification by glycosylation and phosphorylation (88) Baculovirus capsids can package at least two genomic lengths[80], suggesting that it may be possible to insert families of genes, or a large number of genes.

The general strategy involved in the formation of baculovirus recombinants involves: 1) Use of recombinant plasmids containing the Pn gene transcriptional regulatory sequences joined to foreign protein coding sequences, flanked by DNA from a non-essential region of the baculovirus genome, 2) Use of transfection procedures to introduce the plasmid and viral DNA into cells where homologous recombination leads to insertion of the chimeric gene into the same non-essential region of the viral genome, and 3) Isolation and plaque purification of recombinant virus.

In two reports where foreign genes have been inserted for the purposes of expresion in baculovirus infected cells,[81,82] the foreign DNA was inserted into the polyhedrin gene region of AcMNPV such that expression was under the control of the Pn gene promoter and the phenotype of the recombinant was OB⁻. Smith et

al.[81] inserted the human B-interferon gene at a variety of locations with respect to the transcription start site of the Pn gene. The gene was most efficiently expressed when inserted 3 nucleotides upstream from the ATG initiating codon of the Pn gene as opposed to any in-frame or out-of-frame insert within the Pn gene. The study indicated that the gene was expressed abundantly, and the protein was glycosylated and secreted from the infected cells. The interferon was biologically active as determined by a VSV plaque reduction assay.[83] Details on the mechanism of secretion and type of glycosylation remain to be determined.

Miller and colleagues[82] expressed E. coli B-galactosidase as a polyhedrin-fusion protein which resulted in the formation of blue plaques in the presence of X-gal. The specific activity of the galactosidase fusion product was the highest ever reported for crude cell lysates.

Recombinant baculoviruses that express foreign genes in insects or insect tissue culture cells could be constructed to produce protein for a variety of reasons: 1) to produce antigens for use as sub-unit vaccines or for use in clinical diagnosis; 2.) to produce enzymes for therapeutic applications; 3.) to produce proteins which have a toxic effect to invertebrates; 4.) to produce enzymes for use in agricultural or food applications; 5.) to produce active antibody or other multisubunit proteins by expression of each sub-unit polypeptide in the same cell such that the two chains assemble correctly.

One gene of interest is the gene which encodes the surface antigen of hepatis B virus (HBsAg). Hepatitis B virus (HBV) is the causative agent of a disease which is estimated to chronically afflice 200 million persons world wide (7). Since HBV does not propagate in any tissue culture system, nor does it infect convenient experimental animals, the source of this antigen has been confined to the sera of infected individuals (review B). HBsAg represents both the major envelope protein and the neutralizing antigen of the infectious virion and as such has proven effective as a vaccine against the disease.

The HBsAg-gene has been expressed in many different expression vectors with a variety of results. Prokaryotic expression systems yielded only low levels of HBsAg-related immunological material even in systems using powerful bacterial promoters (9, 10). Eukaryotic systems like yeast (11, 12), SV40 based plasmids (13, 14), herpes simplex virus (15), and vaccinia virus (160, produced amounts of HBsAg ranging from 1 mg to 20 mg per liter of culture. Recombinant baculoviruses have the capacity to produce as much as 1 gm of HBsAg per liter of culture (see below.)

Correct folding, hence better presentation of the viral epitope for vaccination which should result in stronger immunity in vaccinated individuals and more thorough protection for vaccinated populations.

Other genes of interest in sub-unit vaccine production would include those derived from viruses such as EBV, AIDS retrovirus, influenza, RSV, etc. Genes derived from these viruses encode proteins that act as antigens in a host immune system such that protective immunity be effected.

The invention concerns a method for producing in a suitable insect host cell a selected polypeptide which comprises isolating a first DNA segment including a viral promoter from a virus capable of infecting said insect host cell, isolating from a suitable source a second DNA segment containing the sequence encoding said selected polypeptide, combining said first and second DNA segments to form a continuous strand of a third DNA segment including vector DNA in which said second DNA segment lies adjacent to and is in-frame with said promoter of said first DNA segment and contains transcription termination signals, forming a recombination vector by effecting recombination between said third segment and baculovirus genomic DNA, contacting said recombination vector so produced with said insect host cell under conditions such that said recombination vector becomes incorporated into said insect host cell to produce an infected insect host cell , growing the host cell so infected and isolating the selected polypeptide from said host cell or culture fluids.

The invention also concerns a recombination vector which comprises a first DNA segment including vector DNA in which a second segment containing the sequence encoding the selected polypeptide lies adjacent to and is in frame with said promoter of said first DNA segment and contains transcription termination signals.

The invention also concerns a polypeptide molecule synthesized in and recoverable from an infected insect cell said polypeptide being actively or passively secreted from the transformed cell and being thereby recoverable.

Also concerned is an infected insect cell which synthesizes and actively or passively secretes a selected polypeptide comprising DNA encoding said polypeptide in frame with a viral promoter and containing transcription termination signals, which DNA is incorporated into said insect host cell so as to produce an infected insect cell such that said polypeptide is synthesized under the control of the viral promoter, said polypeptide being recoverable from the cell or culture fluid.

Further, the invention concerns a method for producing in a suitable insect host cell Hepatitis B virus surface antigen which comprises isolating a first DNA segment including a polyhedrin promoter from a baculovirus capable of infecting said insect host cell, isolating from a suitable source a second DNA segment containing the sequence encoding said surface antigen, combining said first and second DNA segments to form a continuous strand of a third DNA segment including vector DNA in which said second DNA segment lies adjacent to and is in-frame with said promoter of said first DNA segment and contains transcription termination signals, forming a recombination vector by effecting recombination between said third segment and baculovirus genomic DNA, contacting said recombination vector so produced with the insect host cell under conditions such that said recombination vector segment becomes incorporated into said insect host cell to produce an infected host cell, and growing said infected cells under suitable conditions and isolating from the cells or culture fluids the Hepatitis B virus surface antigen so produced.

The invention also concerns a recombination vector which comprises a DNA segment, including baculovirus vector DNA, encoding Hepatitis B virus surface antigen adjacent to and controlled by the polyhedrin promoter and containing transcription termination signals.

The invention also concerns a modified insect host cell which synthesizes Hepatitis B virus surface antigen, produced by infection of said insect cell with a recombination vector including sequences encoding said surface antigen, such that the transcription of said surface antigen is under the control of a polyhedrin promoter in the vector.

Finally, the invention concerns an infected insect cell, which synthesizes and actively or passively secretes hepatitis B virus surface antigen, comprising DNA encoding said surface antigen in frame with the polyhedrin promoter and containing transcription termination signals, such that said surface antigen is synthesized under the control of the polyhedrin promoter, said surface antigen being recoverable therefrom.

DESCRIPTION OF THE FIGURES:

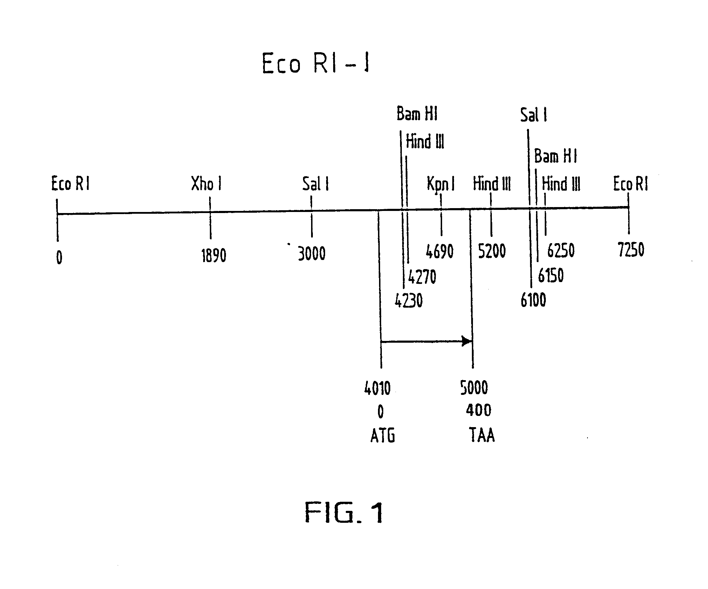

Figure 1.    Restriction map of the EcoRI- I fragment of AcMNPV, strain HR3.

Restriction sites and fragment sizes were derived from Cochran et. al. (89) and Cochran (90). The horizontal line with the arrow indicates the location and polarity of the polyhedrin gene running from its ATG to its TAA codons which correspond to the translational initiation and termination codons, respectively ( 91, 92).

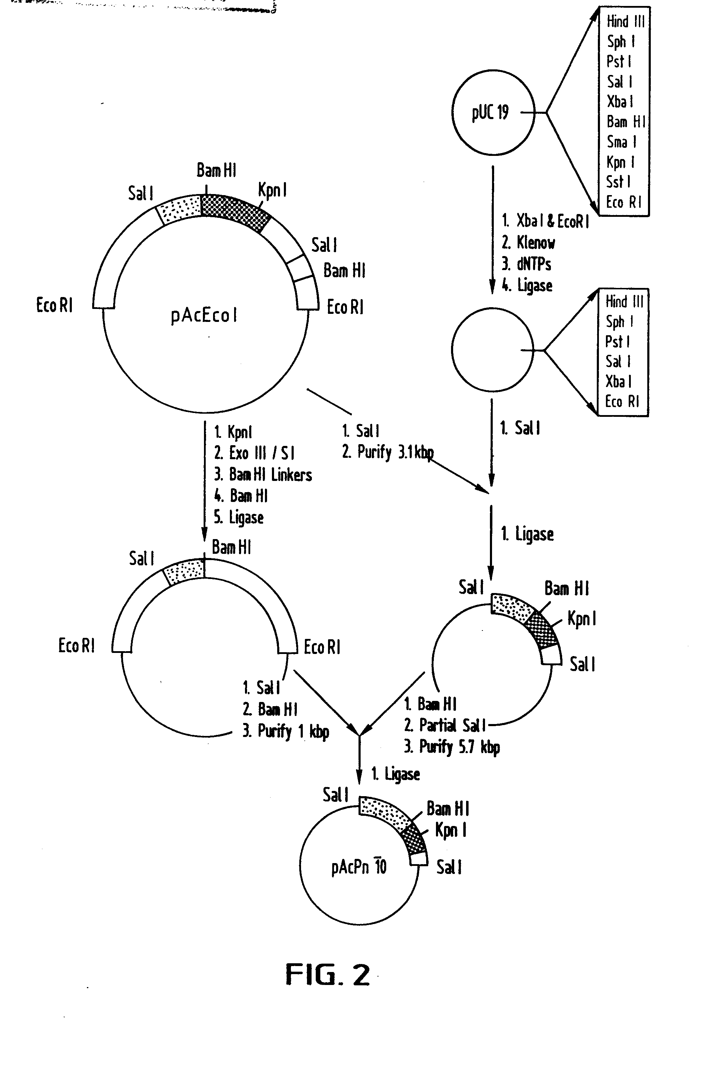

Figure 2.    Construction of a baculovirus insertion vector.

Sequences from pAcEcoI which contain the polyhedrin gene and its controlling elements were treated from the unique KpnI site by treatment with nucleases ExoIII and SI. BamHI linkers were added to the deleted ends and the DNA was digested with BamHI and ligated under dilute conditions such that the distal BamHI site was ligated to the treated ends. SalI/BamHI fragments were isolated from the resultant clones and those that were thought to contain appropriate deletions were inserted in place of the promoter-containing SalI/BamHI fragments of a pUC clone . The resultant recombinants, designated pAcPn series, contained the polyhedrin promoter region with a unique BamHI site downstream from the transcriptional start site. This BamHI site is located about 10 nucleotides upstream from the location of the wild type ATG codon in the insertion vector pAcPn⁻10.

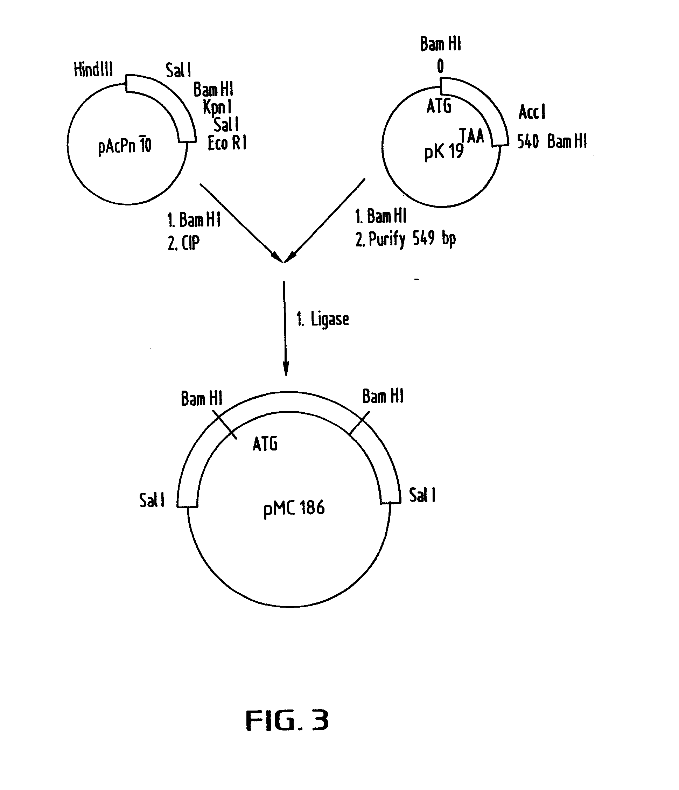

Figure 3.    Construction of VGF recombination vector.
The BamHI fragment of pK19 was inserted into the BamHI site of the pAcPn⁻10 insertion vector. The correct orientation of the VGF gene was determined by the location of the AccI site. The recombination vector with VGF in the correct orientation with respect to the polyhedrin gene was designated pMCI186.

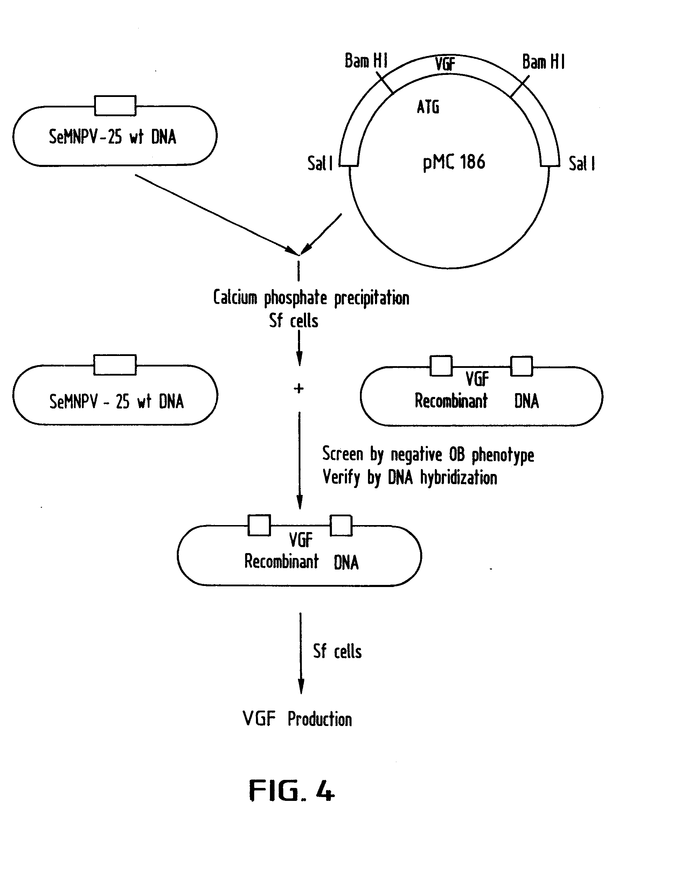

Figure 4.    Preparation , isolation and expression of VGF expressing recombinant baculovirus.

DNA isolated from wild-type SeMNPV-25 and the VGF recombination vector pMC186 were precipitated with calcium phosphate and co-transfected into Spodoptera frugiperda cells. After homologous recombination was allowed to occur, OB⁻ recombinant virus was selected. The presence of VGF sequences was determined by the DNA hybridization using pKl9 as probe. VGF recombinants were plaque purified three times , stock virus was grown up and used to infect large volume cultures of Sf and Tni cells for VGF production.

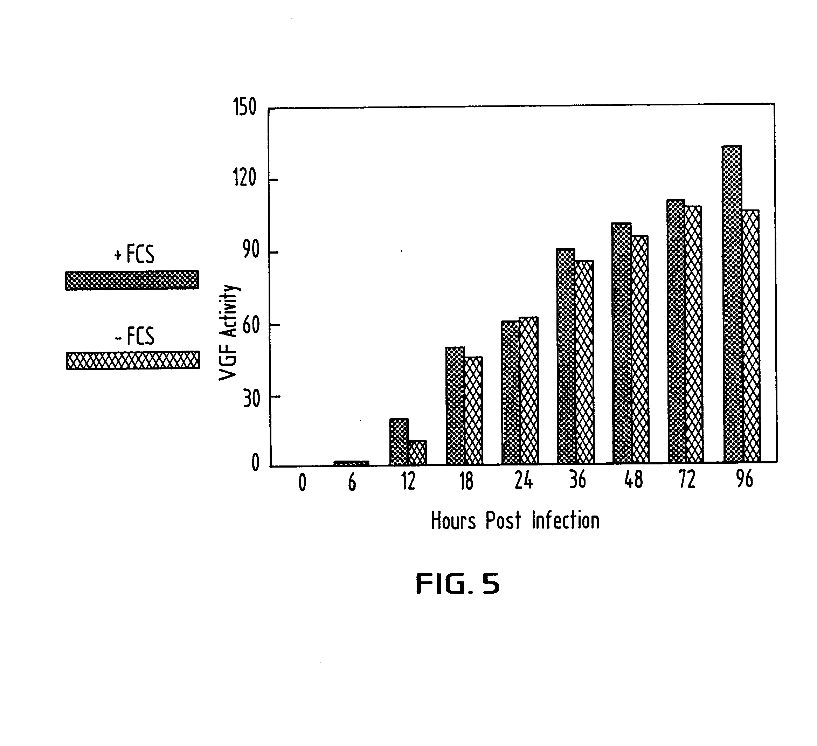

Figure 5.    Time course of VGF production in infected cells in media with and without fetal calf serum.

Replicate cultures of Sf cells were infected with 10 PFU of AcVGF recombinant virus and maintained in the presence (filled bars) and absence (diamond bars) of 10% FCS. At the indicated times after infection, the culture media was harvested and assayed for VGF activity by the radioreceptor assay described previously. VGF activity is expressed as ng/ml of EGF equivalents.

The method according to the present invention may be applied for the production of any polypeptide of interest.

Examples for insect host cells which may be used in the method of the invention are cells of <u>Trichoplusia ni</u>, <u>Spodoptera frugiperda</u>, <u>Heliothis zea</u>,      or <u>Manduca sexta</u>. Preferably, the insect host cell is a caterpillar cell.

Examples for selected polypeptides of interest which may be produced according to the method of the invention are:

Hepatitis B virus surface antigen;
growth factors, like epidermal growth factor, or transforming growth factor;
interferons,
polypeptides derived from the envelope of an AIDS-associated retrovirus;
polypeptides derived from the nucleoprotein core of an AIDS-associated retrovirus;
polypeptides derived from an organism of the genus <u>Plasmodium</u>;
polypeptides derived from the circumsporozooite form of an organism of the genus <u>Plasmodium</u>;
polypeptides derived from the Epstein-Barr virus;
the heavy chain of a human immunoglobulin molecule;
the light chain of a human immunoglobulin molecule;
the heavy chain of a mouse immunoglobulin molecule;
the light chain of a mouse immunoglobulin molecule;
hybrid immunoglobulin molecules, e.g. molecules, wherein the variable region of the hybrid immunoglobulin molecule is derived from a mouse variable region sequence, or molecules, wherein the constant region of the hybrid immunoglobulin molecule is derived from a human constant region;
fusion polypeptides which comprise a hybrid immunoglobulin molecule contiguous with or connected through an amino acid sequence to an enzyme molecule; in said fusion protein the hybrid immunoglobulin molecule may comprise a murine

derived variable region sequence, and the enzyme molecule may be a growth factor, a growth regulator or a urokinase.

Examples for the viral promoters which may be used in the method of the invention are the polyhedrin promoter of baculovirus, the 10k promoter of baculovirus, the granulin promoter of granulosis virus, or a viral promoter which is constructed from synthetic oligomers.

The virus is preferably a baculovirus. Said virus can be isolated e.g. from Autographa californica, from Trichoplusia ni, from Rachiplusia ou, or from Galleria melonella.

<u>Materials and Methods</u>

A.  Cells and Virus:  SeMNPV-25, a genomic variant of AcMNPV was obtained from Dr. D. Knudson (Yale University) and was grown and titred in Sf21-AE cells using TC100 (KC Biologicals) with 10% FCS.

B.  Purification of Viral DNA:  Plaque purified isolates of wild-type and recombinant NPV was grown in Sf cells.  Extracellular nonoccluded virus was isolated, and viral DNA was purified [as described previously (: ).

C.  Construction of Insertion Vector: A series of deletions were constructed from pAcEcoI (89) by exonuclease III digestion followed by nuclease SI digestion as described by Tamanoi and Stillman, (93) and modified by Cochran et al.(86).

The Eco-Rl fragment of AcMNPV-HR3 DNA was cloned into a pUC vector.  A 50 ug sample of pAcEcoI was linearized at its unique KpnI site and digested with 100U of exonuclease III in 6.6mM Tris-Hcl (pH 7.4) – 6.6 mM $MgCl_2$ – 6.6 mM 2-mercaptoethanol and – 50mM naCl.  After 5 min of incubation at $18^{\circ}C$, equal volumes of 2X concentrated S1 buffer and 600U of nuclease S1 were added and the DNA was incubated for three hours at $18^{\circ}C$.  Under these conditions up to 700 bp were removed from each KpnI terminus.  After treatment with the Klenow fragment of DNA polymerase I to ensure that all the ends were flush, BamHI linkers were blunt-end ligated to the DNA.  After digestion with an excess of BamHI, the plasmid was recircularized by ligtation under dilute conditions.

The deletion end-points of each selected mutant were deduced from the electrophoretic mobility of the SalI - BamHI fragments on polyacrylamide gels.

Another plasmid was constructed by insertion of the SalI fragment, derived from the pAcEcoI, into the SalI site of a pUC19 plasmid which had a multiple cloning site deleted between the XhoI and EcoRl sites.

Sal I/BamHl fragments were isolated from the pAcEcoI deletion mutants and those in the range of 1000 bp were inserted in place of the promoter-containing Sal I/BamI fragments of the pUC clone.

The resultant recombinants, designated pAcPn series, contained the polyhedrin promoter region with a unique BamHl site downstream from the transcriptional start site. In the recombinant designated pAcPn⁻10, this BamHl site is located about 10 nucleotides upstream from the location of the wild-type ATG codon.

D. Construction of Baculovirus Recombinants that Express VGF: A 540 bp DdeI fragment from the vaccinia virus 19K early gene region was isolated and BamHI linkers were added prior to insertion into pUC18. The resultant plasmid was designated pK19. The DdeI fragment contains the entire VV gene which encodes the 19K protein that has homology with EGF and TGF. The BamHI fragment of pK19 was isolated from pK19 and cloned into the BamHI site of the

baculovirus insertion vector pAcPn-10. Recombinant plasmids containing the 19K insert were analysed by restriction endonuclease digestions to confirm their proper orientation.

The chimeric VGF gene was inserted into the SeMNPV-25 genome by homologous recombination. Briefly, 20 ug of the recombinant plasmid and 1 ug of SeMNPV-25 DNA were gently mixed in 1 ml of Hepes-buffered saline (NaCl, 0.14M; KCl, 5mM; $Na_2HPO_4 \cdot 2H_2O$, 1mM; Dextrose, 0.1%;Hepes, 20mM; pH 7.05). A 50 ul volume of 2.5 M $CaCl_2$ was slowly added and gently mixed. The mixture was left at room temperature for 15 min during which time a fine white precipitate formed. This mixture was added to semi-confluent Sf cells in a $25cm^2$ flask. This was gently rocked for 30 in at room temperature and 5 ml of prewarmed TC-100 was added to the cells. The infected cell supernatant was harvested at 72 hr post infection, diluted to the $10^{-5}$ range and plaqued in Sf cells. At about four days post-infection recombinant virus infected cells could be identified under the microscope as plaques with identifiable cytopathic effect but no nuclear occlusions. Several plaques were picked and two further plaque purifications were carried out to obtain pure recombinant virus. DNA:DNA hybridization using pK19 as a probe was used to help in the identification of recombinants.

## Results and Discussion

Construction of Insertion Vector: Mocarski et al. (95) demonstrated the utility of homologous recombination for the insertion of any DNA fragment into viral genomes when the fragment is flanked by homologous sequences from the virus.

Several groups have described the expression of foreign genes from transcriptional regulatory regions of vector genomes after insertion of chimeric genes by homologous recombination. We chose to apply these examples for expression of foreign genes in baculovirus infected cells. The first task was to construct a generalized cloning vector which contained unique cloning sites at a position downstream from a defined baculovirus promoter, all of which would be flanked by sequences homologous to the viral genome so as to direct insertion by homologous recombination.

We constructed an insertion vector as described in Figure 1 and in Materials and Methods. Briefly, deletions in the polyhedrin gene containing EcoRl -I fragment were introduced at the KpnI site and BamHl linkers were added to the deletion end-points. A recombinant plasmid, designated pAcPn-10, was isolated which contained a unique BamHl site at a position 10 base pairs upstream from the deleted ATG initiation codon of the polyhedrin gene.

Foreign sequences introduced at this site in the proper orientation will be expressed from the polyhedrin promoter and the transcript will be translated into an authentic polypeptide providing it contains its own ATG and TAA translational initiation and termination codons, respectively.

The insertion vector pAcPn-10 represents a first generation vector. Subsequent vectors will include an assortment of the following features: a) insertion sites in addition to the BamHl site; b) unique restriction sites for fragments that include the promoter and ATG codon of the baculovirus gene used such that foreign genes could be ligated in frame; c) additional regulatory sequences, such as a repeat region of AcMNPV; d) signal sequences that would allow for the active secretion of the foreign gene product; e) other baculovirus promoter sequences; and f) synthetic oligomers designed to be active in a baculovirus infected cell.

## Construction of Baculovirus recombinants that express VGF.

The 540 base pair fragment from pK19 which contains the complete coding region of VGF was isolated from pK19 as a BamHl fragment inserted into the BamHl site of pAcPn-10. The orientation of the gene was confirmed by the use of the AccI site located at position 419 and the plasmid with the correct orientation was designated as pMC160. This is detailed in Fig. 2.

As described in Figure 3, recombinant virus was prepared by co-transfecting Sf cells with calcium phosphate-precipitate wild-type SeMNPV-25 DNA and pMC160.

The cells were harvested and OB⁻ plaques were isolated from plaque assay plates. Virus in plaques was then screened for the presence of VGF DNA by dot-blot hybridication using pK19 as a probe. Of 24 plaques picked 6 indicated the presence of VGF DNA. These were plaqued 20 more times and again were verified to be OB⁻ and VGF- DNA+. Large stocks of the recombinant viruses were prepared in Sf cells.

To confirm the predicted structures of the recombinant virus, DNA was purified from NOV derived from recombinant and wild-type infected cells and their restriction fragment profiles were compared.

Expression of VGF recombinant baculoviruses.

To assay for the expression of the VGF gene, monolayers of Sf cells were infected with about 10 PFU per cell of recombinant virus and samples of cells and cell culture medium harvested at various times after infection. Quantitation of VGF expression is detailed in Table 2.

TABLE 2

VGF Production in Insect Cells

| Sample | VGF Activity, ng. of EGF per $10^6$ cells |
|---|---|
| uninfected T.ni cells | 0.0 |
| uninfected T.ni cell media | 0.0 |
| wt. infected T.ni cells | 0.0 |
| wt. infected T.ni. cell media | 0.0 |
| AcVGF infected T.ni. cells | 140.0 |
| AcVGF infected T.ni cell media | 125.0 |
| AcVGF infected S.f. cells | 200.0 |
| AcVGF infected S.f. cell media | 6.5 |

Replicate cultures of 1 x $10^6$ T.ni.and S.f.. cells were infected with wild type SeMNPV-25 or AcVGF recombinant virus. The multiplicity of infection was 10 PFU. The cells were harvested at 72 hours post infection along with uninfected cells. Both cell lysates and cell culture supernatant were assayed for VGF activity as expressed as EGF equivalents.

The proteins contained in the samples were analyzed by SDS- polyacrylamide gel electrophoresis and then assayed for their ability to inhibit the binding of mouse $^{(125)}$I-EGF to Swiss 3T3 cell.

Polyacrylamide gels indicated the presence of an additional gene product at about 18K in the samples infected with the recombinant at times beyond 12 hpi. This 18K gene product was present in both the cells and the cell culture media.

Cell culture media was then assayed for EGF activity. Samples infected with recombinant virus at 12 hours onward were found to contain increasing amounts of material· that competed with mouse $^{125}$I-EGF for the receptor on 3T3 cells.

To test for expression of the VGF gene without the addition of FBS to incomplete media, replicate cultures of Sf cells were infected with recombinant virus and maintained in media with or without the addition of FBS. At various times up to 96 hours after infection the cell culture media was harvested and analyzed in the competitive assay. Figure 6 indicates that near equivalent amounts of activity are contained in each sample up to 72 hpi when expression in samples without FBS has leveled off while expression in samples with FBS has not. This experiment indicates that VGF recombinant product can be produced in and be purified from otherwise protein free media.

A 50 ml volume of recombinant infected cell culture media without FBS was used for purification of the $^{125}$I-EGF competing material. More than 1 mg, of an 18K polypeptide was purified by HPLC. This polypeptide was found to co-migrate on SDS-polyacrylamide gels with the additional 18K polypeptide identified in infected cells.

The VGF purified from cells infected with vaccinia virus is glycosylated and has an apparent molecular weight of 23,000 (94). The results presented herein suggest that VGF as expressed in the baculovirus system is not fully glycosylated and has a molecular weight of approximately 18K, but retains its activity. Hence, the additional size of the authentic VGF product does not appear to be necessary for its activity.

In accordance with the invention, a variety of medically and commercially important polypeptides and proteins may be produced. As disclosed herein, methods of the invention may be used to produced Hepatitis B surface antigen. Growth factors, such as vaccinia virus growth factor, epidermal growth factor and transforming growth factor, and polypeptides such as AIDS retrovirus envelope and core antigens, malaria sporozoite antigen, Epstein-Barr virus membrane surface antigen, respiratory syncytial virus antigen and parainfluenza virus antigen may be produced.

Further, receptor binding or receptor effecting proteins, such as protein sweeteners, neuropeptides and growth regulatory factors, may be produced using the methods of this invention.

The methods of this invention allow modification of selected polypeptides to produce analogs with increased beneficial bioactivity. Further, this invention allows production of specific antigenic polypeptides for use in vaccines. The antigenic polypeptides may be engineered such that amino acid sequences encoding all or part of other polypeptide molecules at the amino or carboxy-terminal region of the subject antigenic polypeptides. Thus, these amino or carboxy-terminal portions may function as adjuvants or immunopotentiators in the vaccine.

Further, using the disclosed methods, heavy and light chain subunits of specific immunoglobulin molecules may be produced in the same insect cell, either by co-infection with plasmids encoding heavy and light subunits or by infection with one plasmid which encodes both the heavy and light chains in its sequence. Similarly, other multisubunit molecules, such as insulin, may be produced.

As will be apparent to those skilled in the art in the light of the foregoing disclosure, many modifications, alterations and substitutions are possible in the practice of this invention without departing from the spirit or scope thereof.

## References

1. Mathews, R.E., Classification and nomenclature of viruses, Fourth Report of the International Committee on Taxonomy of Viruses. Intervirology, 17, 1, 1982.

2. Smith, F.E., Summers, M.D., Analysis of baculovirus genomes with restriction endonucleases. Virology, 89, 517, 1978.

3. Miller, L.K., and Dawes, K.P., Physical map of the DNA genome of Autographa californica nuclear polyhedrosis virus, J. Virol., 29, 1044, 1979.

4. Tjia, S.T., Carstens, E.B., and Doerfler, W., Infection of Spodoptera frugiperda cells with Autographa californica nuclear polyhedrosis virus. II. The viral DNA and kinetics of its replication. Virology, 99, 399, 1979.

5. Cochran, M.A., Carstens, E.B., Eaton, B.T., and Faulkner, P., Molecular cloning and physical mapping of restriction endonuclease fragments of Autographa californica nuclear polyhedrosis virus DNA. J. Virol., 41, 940, 1982.

6. Smith, G.E., and Summers, M.D., Restriction maps of five Autographa californica MNPV variants, Trichoplusia ni MNPV, and Galleria mellonella MNPV DNAs with endonucleases SmaI, KpnI, BamHI, SacI, XhoI, and EcoRI. J. Virol., 30, 828, 1979.

7. Brown, S.E., Maruniak, J.E., and Knudson, D.L., Physical map of SeMNPV Baculovirus DNA: An AcMNPV Geonomic Variant. Virology, 136, 235, 1984.

8. Smith, G.E., and Summers, M.D., Restriction map of Rachiplusia ou and Rachiplusia ou - Autographa californica baculovirus recombinant. J. Virol., 33, 311, 1980.

9. Jewell, J.E., and Miller, L.K., DNA sequence homology relationships among six lepidopteran nuclear polyhedrosis viruses. J. Gen. Virol., 48, 161, 1980.

10. Nathans, D., and Smith, H.O., Restriction endonucleases in the analysis and restructuring of DNA molecules. Annual Rev. biochem, 44, 273, 1975.

11. Lubbert, H., Kruczek, I., Tjia, S., and Doerfler, W., The cloned EcoRI fragments of Autographa californica nuclear polyhedrosis virus DNA. Gene, 16, 343, 1981.

12. Vlak, J.M., Mapping of BamHI and SmaI DNA restriction sites on the genome of the nuclear polyhedrosis virus of the alfalfa looper, Autographa californica. J. Invertebr. Pathol., 36, 409, 1980.

13. Evanson, D.P., Electron microscopy of viral nucleic acids. In "Methods in Virology", K. Maramorsch and H. Kprowski, Eds., Vol. VI, Academic Press, NY., pp. 220, 1977.

14. Southern, E.M., Measurement of DNA length by gel electrophoresis. Anal. Biochem., 100, 319, 1979.

15. Danna, K.J., Determination of fragment order through partial digests and multiple enzyme digests. In "Methods in Enzymology", L. Grossman and K. Moldave, Eds., Academic Press, New York., pp. 449, 1980.

16. Smith, H.O., and Bernstiel, M.L., A simple method for DNA restriction site mapping. Nucleic Acids Res., 3, 2387, 1976.

17. Sata, S., Hutchison, C.A., III, and Harris, J.I., A thermostable sequence-sequence endonuclease from Thermus aquaticus. Proc. Natl. Acad. Sci. (USA), 74, 542, 1977.

18. Legerski, R.J., Hodnett, H.L., and Gray, H.B., Jr., Extracellular nucleases of pseudomonas Bal31. III. Use of the double-stranded deoxyribonuclease activity as the basis of a convenient method for the mapping of fragments of DNA produced by cleavage with restriction enzymes. Nucleic Acids Res., 5, 1445, 1978.

19. Vlak, J.M., and Smith, G.E., Orientation of the genome of Autographa californica nuclear polyhedrosis virus: A proposal. J. Virol., 41, 1118, 1982.

20. Lubbert, H., and Doerfler, W., Mapping of early and late transcripts encoded by the Autographa californica nuclear polyhedrosis virus genome: Is viral RNA spliced?: J. Virol., 50, 497, 1984.

21. Holford, T.R., Brown, S.E., and Knudson, D.L., Estimation of DNA fragment size and generation of DNA restriction endonuclease maps using linear models. J. Virological Methods, 10, 117, 1985.

22. Maruniak, J.E., Brown, S.E., and Knudson, D.L., Physical maps of SfMPV baculovirus DNA and its genomic variants. Virology, 136, 221, 1984.

23. Knell, J.D., and Summers, M.D., Investigation of genetic heterogeneity in wild isolates of Spodoptera frugiperda nuclear polyhedrosis virus by restriction endonuclease analysis of plaque-purified variants. Virology, 112, 190, 1981.

24. Loh, L.C., Hamm, J.J., and Huang, E.S., Spodoptera frugiperda nuclear polyhedrosis virus genome: Physical maps for restriction endonucleases BamHI and HindIII. J. Virol., 38, 922, 1981.

25. Arif, B., Kuzio, J., Faulkner, P., and Doerfler, W., The genome of Chorisoneura fumiferana nuclear polyhedrosis virus: Molecular cloning and mapping of the EcoRI, BamHI, SmaI, XbaI and BgIII restriction sites. Virus Res., 1, 605, 1984.

26. Knell, J.D., and Summers, M.D., A physical map for the Heliothis zea SNPV genome. J. gen. Virol., 65, 445, 1984.

27. Leisy, D.J., Rohrmann, G.F., and Beaudreau, G.S., Conservation of genome organization in two multicapsid nuclear polyhedrosis viruses. J. Virol., 52, 699, 1984.

28. Smith, G.E., Vlak, J.M., and Summers, M.D., In vitro translation of Autographa californica nuclear polyhedrosis virus early and late mRNAs. J. Virol., 199, 1982.

29. Cochran, M.A., and Faulkner, P., Location of homologous DNA sequences interspersed at five regions in the baculovirus AcMNPV genome, J. Virol., 45. 961, 1983.

30. Arif, B.M., and Doerfler, W., Identification and localization of reiterated sequences in the Choristoneura fumiferana MNPV genome. Eur. Mol. Biol. Organ. J., 3, 525, 1984.

31. Smith, G. E., and Summers, M.D., DNA homology among subgroup A, B, and C baculoviruses. Virology, 123, 393, 1982.

32. Howley, P. M., Israel,l M.A., Law, M., and Martin., A rapid method for detecting and mapping homology between heterologous DNAs, J. Biol, Chem., 254, 4876, 1979.

33. Yang, R.C. Young, S., and Wu, R., BK virus DNA sequence coding for t and T antigens and evaluation of methods for determining sequence homology. J. Virol., 34, 416, 1980.

34. Kelly, D. C., The DNA contained by nuclear polyhedrosis viruses isolated from four Spodoptera sp. (Lepidoptera, Noctuidae): Genome size and homology assessed by DNA reassociation kinetics. Virology, 76, 468, 1977.

35. Knudson, D. L., and Tinsley, T. W., Replication of a nuclear polyhedrosis virus in a continuous cell line of Spodoptera frugiperda: Partial characterization of the viral DNA, comparative DNA-DNA hybridization, and patterns of DNA synthesis. Virology, 87, 42, 1978.

36. Rohrman, G.F., Martignoni, M.E., and Beaudreau, G.S., Quantificationn of two viruses in technical preparations of Orgyia pseudotsugata baculovirus by means of buoyant density centrifugation of viral deoxyribonucleic acid. Appl. Environ. Microbiol., 35, 690, 1978.

37. Jurkovica, M., Van Touw, J. S., Sussenbach, J.S., and Schegget, J., Characterization of the nuclear polyhedrosis virus DNA of Adoxophes orana and Borathra brassicae. Virology, 93, 8, 1979.

38. Rohrmann, G. F., Martignoni, M.E., and Beaudreau, G. S., DNA sequence homology between Autographa californica and Orgyia pseudotsugata nuclear polyhedrosis viruses. J. Gen. Virol., 62, 137, 1982.

39. Summers, M. D., Smith, G. E., Knell, J. D., and Burand, J. P., Physical maps of Autographa californica and Rachiplusia ou nuclear polyhedrosis virus recombinants. J. Virol., 34, 693, 1980

40. Rohel, D., Z., Chochran, M. A., and Faulkner, P., Characterization of two abundant mRNAs of Autographa californica nuclear polyhedrosis virus present late in infection. Virology, 124, 357, 1983.

41. Smith, G. E., Vlack, J. M., and Summers, M. D., Physical analysis of Autographa californica nuclear polyhedrosis virus transcripts for polyhedrin and 10,000 molecular weight protein. J. Virol., 45, 215, 1983.

42. Arif, B. M., Tjia, S. T., and Doerfler, W., DNA homologies between the genomes of Choristoneura fumiferana and Autographa californica nuclear polyhedrosis viruses. Virus Res., 2, 85, 1985.

43. Singh, S. P., Gudauskas, R. T., and Harper, J D., High resolution two-dimensional gel electrophoresis of structural proteins of baculoviruses of _Autographa californica_ and _Porthetria_ (_Lymantria_) _dispar_. Virology, 125, 370, 1983.

44. Singh, S. P., Gudauskas, R. T., Harper, J. D., and Edwards, J., Two-dimensional gel electrophorsis of basic proteins of _Autographa californica_ nuclear polyhedrosis virus. J. Invertebr. Pathol., 45, 249, 1985.

45. Carstens, E. B., Tjia, S. T., and Doerfler, W., Infection of _Spodoptera frugiperda_ cells with _Autographa californnica_ nuclear polyhedrosis virus. I. Synthesis of intracellular proteins after virus infection. Virology, 99, 386, 1979.

46. Dobos, P., and Cochran, M. A., Protein synthesis in cells infected by _Autographa californica_ nuclear polyhedrosis virus (AcNPV): The effect of cystosine arabinoside. Virology, 103, 446, 1980.

47. Maruniak, J. E., and Summers, M. D., _Autographa californica_ nuclear polyhedrosis virus phosphoproteins and synthesis of intracellular proteins after virus infection. Virology, 109, 25, 1981.

48. Wood, H. A., _Autographa californica_ nuclear polyhedrosis virus induced proteins in tissue culture. Virology, 102, 21, 1980.

49. Kelly, D. C., and Lescott, T., Baculovirus replication: Protein synthesis in _Spodoptera frugiperda_ cells infected with _Trichoplusia ni_ nuclear polyhedrosis virus. Microbiologica, 4, 35, 1981.

50. Vlak, J. M., Smith, G. E., and Summers, M. D., Hybridization selection and in vitro translation of _Autographa californica_ nuclear polyhedrosis virus mRNA. J. Virol., 40, 762, 1981.

0228036

.51. Miller, L. K., Trimarchi, R. E., Browne, D., and Pernnock, G. D., A temperature-sensitive mutant of the baculovirus <u>Autographa</u> <u>californica</u> nuclear polyhedrosis virus defective in an early function required for further gene expression. Virology, 126, 376, 1983.

52. Jun-Chuang, Q., and Weaver, R. F., Capping of viral RNA in cultured <u>Spodoptera</u> <u>frugiperda</u> cells infected with <u>Autographa</u> <u>californica</u> nuclear polyhedrosis virus. J. Virol., 43, 234, 1982.

53. Banerjee, A. K., 5' terminal cap structure in eukaryotic messenger ribonucleic acids. Microbiol. Rev., 44, 175, 1980.

54. Van Der Beek, C. P., Saaijer-Riep, J. D., and Vlak, J. M., On the origin of the polyhedral protein of <u>Autographa</u> <u>californica</u> nuclear polyhedrosis virus. Isolation, characterization, and translation of viral messenger RNA. Virology, 100, 326, 1980.

55. Vlak, J. M., and Van Der Krol, S., Transcription of the <u>Autographa</u> <u>californica</u> nuclear polyhedrosis virus genome: Location of late cytoplasmic mRNA. Virology, 123, 222, 1982.

56. Brula, M. A., Buller, P. L., and Weaver, R. F., Alpha-amanitin-resistant viral RNA synthesis in nuclei isolated from nuclear polyhedrosis virus-infected <u>Heliothis</u> <u>zea</u> larvae and <u>Spodoptera</u> <u>frugiperda</u> cells. J. Virol., 38, 916, 1981.

57. Brown, M., Crawford, A. M., and Faulkner, P., Genetic analysis of a baculovirus, <u>Autographa</u> <u>californica</u> nuclear polyhedros. I. Isolation of temperature-sensitive mutants and assortment into complementation groups. J. Virol., 31, 190, 1979.

58. Lee, H. H., and Miller, L. K., Isolation, complementation, and initial characterization of temperature-sensitive mutants of the baculovirus Autographa californica nuclear polyhedrosis virus. J. Virol., 31, 240, 1979.

59. Duncan, R., and Faulkner, P., Bromodeoxyuridine-induced mutants of Autographa californica nuclear polyhedrosis virus defective in occlusion body formation. J. gen. Virol., 62, 369, 1982.

60. Miller, L. K., Construction of a genetic map of the baculovirus Autographa californica nuclear polyhedrosis virus by marker rescue of temperture-sensitive mutants. J. Virol., 39, 973, 1981.

61. Potter, K. N., and Miller, L. K., Transfection of two invertebrate cell lines with DNA of Autographa californica nuclear polyhedrosis virus. J. Invertebr. Pathol., 36, 431, 1980

62. Adang, M. J., and Miller, L. K, Molecular cloning of DNA complementary to mRNA of the baculovirus Augtographa californica nuclear polyhedrosis virus: Location and gene products of RNA transcripts found late in infection. J. Virol., 44, 782, 1982.

63. Esche, H., Lubbert, H., Siegmann, B., and Doerfler, W., The translational map of the Autographa californica nuclear polyhedrosis virus (AcNPV) genome. Eur. Mol. Biol Organ. J., 1, 1629, 1982.

64. Rohel, D. Z., Cochran, M. A., and Faulkner, P., Mapping late mRNA populations of Autographa californica nuclear polyhedrosis virus by 'criss-cross' DNA-RNA hybridization. J. gen. Virol., 65, 809, 1984.

65. Erlandson, M. A., and Carstens, E. B., Mapping early transcription products of Autographa californica nuclear polyhedrosis virus. Virology, 126, 398, 1983.

66. Lubbert, H., and Doerfler, W., Transcription of overlapping sets of RNAs from the genome of Autographa californica nuclear polyhedrosis virus: A novel method for mapping RNAs. J. Virol., 52, 255, 1984.

67. Friesen, P. D., and Miller, L. K., Temporal regulation of baculovirus DNA: Overlapping early and late transcripts. J. Virol., 54, 392, 1985.

68. Baty, D., Barrera- Saldanna, H. A., Everett, R. D., Vigneron, M., and Chambon, P., Mutational dissection of the 21 bp repeat region of the SV40 early promoter reveals that it contains overlapping elements of the early-early and late-early promoters. Nucleic Acids Res., 12,

69. Buchman, A. R., Fromm, M., and Berg, P., Complex regulation of simian virus 40 early-region transcription from different overlapping promoters. Mol. Cell. Biol., 4, 1900, 1984.

70. Cochran, M. A., Fuckett, C., and Moss, B., In vitro mutagenesis of the promoter region for vaccinia virus gene: Evidence for tandem early and late regulatory signals. J. Virol., 54, 30, 1985.

71. Bajszar, G., Wittek, R., Weir, J. P., and Moss, B., Vaccinia virus thymidine kinase and neighboring genes: MRNAs and polypeptides of wild-type virus and putative nonsense mutants. J. Virol., 45, 62, 1983.

72. Hooft Van Iddekinge, B. J., Smith, G. E., and Summers, M. D., Nucleotide sequence of the polyhedrin gene of Autographa californica nuclear polyhedrosis virus. Virology, 131, 561, 1983.

73. Rohrmann, G. F., Leisy, D. J., Chow, K. C., Pearson, G. D., and Beaudreau, G. S., Identification, cloning, and R-loop mapping of the polyhedrin gene from multicapsid nuclear polyhedrosis virus of Orgyia pseudotsugata. Virology, 121, 51, 1982.

74. Kozlov, E. A., Levitina, T. L., Gusak, N. M., and Serebryani, S. B., Comparison of the amino acid sequence of inclusion body proteins of nuclear polyhedrosis viruses Bombyx mori, Porthetria dispar and Galleria mellonella. Bioorgan. Chem., 7, 1008, 1981.

75. Chakerian, R., Rohrmann, G. F., Nesson, H. M., Leisy, d. J., and Beaudreau, G. S., The nucleotide seqence of the Pieris brassicae granulosis virus granulin gene. J. gen. Virol., 66, in press, 1985.

76. Iatrou, K., Ito, K., and Witkiewicz, H., Polyhedrin gene of Bombyx mori nuclear polyhedrosis virus. J. Virol., 54, 436, 1985.

77. Proudfoot, N. J., and Brownlee, G. G., 3' non-coding sequences in eukaryotic messenger RNA. Nature, 263, 211, 1976.

78. Quant, R. L., Pearson, M. N., Rohrmann, G. F., and Beaudreau, G. S., Production of polyhedrin monoclonal antibodies for distinguishing two Orgyia pseudotsugata baculoviruses. Appl. Environ. Microbiol., 48, 732, 1984.

79. Tjia, S. T., Meyer Zu Altenschildesche, G., and Doerfler, W., Autographa californica nuclear polyhedrosis virus (AcNPV) DNA does not persist in mass cultures of mammalian cells. Virology, 125, 107, 1983.

80. Skuratovskaya, I. Strokovskaya, L. I., Zherebtsova, E. N., and Gudz-Gorban, A. P., Properties of the nuclear polyhedrosis virus of the greater wax moth: Oligomeric circular DNA and the characteristics of the genome. Virology, 120, 465, 1982.

81. Smith, G. E., Summers, M. D., and Fraser, M. J., Production of human beta interferon in insect cells infected withh a baculovirus expression vector. Mol. Cell. Biol., 3, 2156, 1983.

82. Pennock, G. D., Shoemaker, C., and Milelr, L. K., Strong and regulated expression of Escherichia coli beta- galactosidase in insect cells with a baculovirus vector. Mol. Cell. Biol., 4, 399, 1984.

0228036

83. Langford, M. P., Weigent, D. J., Stanton, F. J., and Baron, S., Virus plaque-reduction assay for interferon: Microplaque and regular macroplaque reduction assays. Methods Enzymol., 78, 339, 1981.

84. Carstens, E. B., Tjia, S. T., and Doerfler, W., Infectious DNA from *Autographa californica* nuclear polyhedrosis virus. Virology, 101, 311, 1980.

85. Smith, G. E., and Summers, M. D., Application of a novel radioimmunoassay to identify baculovirus structural proteins that share interspecies antigenic determinants. J. Virol., 39, 125, 1981.

86. Cochran, M.A., S. E. Brown and D.L. Knudson, 1986. Organization and expression of the baculovirus genome. In "Biology of Baculoviruses, I. Biological Properties and Molecular Biology" R.R. Granados and B.A. Federici, EDS., CRC press, Boca Raton.

87. Volkman, L.E. and D.L. Knudson. 1986. *In vitro* replication of baculoviruses. In "Biology of Baculoviruses, I. Biological Properties and Molecular Biology" R.R. Granados and B.A. Federici, EDS., CRC press, Boca Raton.

88. Maruniak, J.E. 1986. Baculovirus structural proteins and protein synthesis. In "Biology of Baculoviruses, I. Biological Properties and Molecular Biology" R.R. Granados and B.A. Federici, EDS., CRC press , Boca Raton.

89. Cochran, M.A., E.B. Carstens, B.T. Eaton and P. Faulkner. 1982. Molcular cloning and physical mapping of restriction fragments of *Autographa californica* nuclear polyhedrosis virus DNA. J. Virol. 41: 940-946.

90. Cochran, M.A. 1982. Physical and functional analysis of the genome of _Autographa californica_ nuclear polyhedrosis virus. PhD Thesis, Queen's University, Kingston, Canada.

91. Rohel, D.Z., M.A. Cochran and P. Faulkner. 1984. Mapping late mRNA populations of _Autographa californica_ nuclear polyhedrosis virus by 'criss-cross' DNA-RNA hybridization. J. gen Virol. 65: 809-813.

92. Rohel, D.Z., M. A. Cochran and P. Faulkner. 1983. Characterization of two abundant mRNAs of _Autographa californica_ nuclear polyhedrosis virus present late in infection. Virology 124: 357-365.

93. Tamanoi, F. and B.W. Stillman. 1983. Initiation of adenovirus DNA replication _in vitro_ requires a specific DNA sequence. Proc. Natil. Acad. Sci. U.S.A. 80: 6446-6450.

94. Stroobant, P., A.P. Rice, W.J. Gullick, D.K. Cheng, I.M. Kerrr and M. Waterfield. 1985. Purification and characterization of vaccinia virus growth factor. Cell 42: 383-393.

95. Mocarski, E.S., L.E. Post and B. Roizman. 1980. Molecular engineering of the herpes virus genome: insertion of a second L-S junction into the genome causes additional genome inversions. Cell 22: 243-255.

96. Burges, H.B., G. Crozier and J. Huber, 1980. A review of safety tests on baculoviruses. Entomophaga 25:329.

0228036

97. Miltenburger, H.G. and A. Krieg, 1984. Bioinsecticides:II Baculoviridae. In "Advances in Biotechnological Processes 3." Alan R. Liss, Inc., New York, pp. 291-313.

C L A I M S

1.  A method for producing in a suitable insect host cell a selected polypeptide which comprises:

isolating a first DNA segment including a viral promoter from a virus capable of infecting said insect host cell;

isolating from a suitable source a second DNA segment containing the sequence encoding said selected polypeptide;

combining said first and second DNA segments to form a continuous strand of a third DNA segment including vector DNA in which said second DNA segment lies adjacent to and is in-frame with said promoter of said first DNA segment and contains transcription termination signals;

forming a recombinant vector by effecting recombination between said third segment and baculovirus genomic DNA;

contacting said recombinant vector so produced with said insect host cell under conditions such that said recombinant vector becomes incorporated into said insect host cell to produce an infected insect host cell;

growing the host cells so infected and isolating the selected polypeptide from said host cells or culture fluids.

2. A recombinant vector which may be used in the method of claim 1, which comprises a first DNA segment including a vector DNA in which a second segment containing the sequence encoding the selected polypeptide lies adjacent to and is in frame with said promoter of said first DNA segment and contains transcription termination signals.

3. An infected insect cell comprising the recombinant vector of claim 2, which synthesizes and actively or passively secretes a selected polypeptide comprising DNA encoding said polypeptide in frame with a viral promoter and containing transcription termination signals, which DNA is incorporated into said insect host cell so as to produce a infected insect cell such that said polypeptide is synthesized under the control of the viral promoter, said polypeptide being recoverable therefrom.

4. A polypeptide produced by the method of claim 1.

5. A polypeptide molecule of claim 4 , wherein said polypeptide is actively or passively secreted from the infected cell and is thereby recoverable.

6. A method for producing in a suitable insect host cell Hepatitus B virus surface antigen which comprises:

isolating a first DNA segment including a polyhedrin promoter from a baculovirus capable of infecting said insect host cell;

isolating from a suitable source a second DNA segment containing the sequence encoding said surface antigen;

by combining said first and second DNA segments to form a continuous strand of a third DNA segment including vector DNA in which said second DNA segment lies adjacent to and is in-frame with said promoter of said first DNA segment and contains transcription termination signals;

forming a recombinant vector by effecting recombination between said third segment and baculovirus genomic DNA;

contacting said recombinant vector so produced with the insect host cell under conditions such that said recombinant vector becomes incorporated into said insect host cell to produce an infected host cell;

growing said infected cells under suitable conditions and isolating from the cells or culture fluids the Hepatitis B virus suface antigen so produced.

7.  A recombinant vector which may be used in the method of
    claim 6 which comprises a first DNA segment including
    baculovirus vector DNA, in which a second segment containing the sequence encoding Hepatitis B virus surface antigen lies adjacent to and is in-frame with the polyhedrin
    promoter and contains transcription termination signals.

8.  A modified insect host cell comprising the recombinant
    vector of claim 7, which synthesizes Hepatitis B virus
    surface antigen, produced by infection of said insect
    cell with a recombinant vector including sequences encoding
    said surface antigen, such that the transcription
    of said surface antigen is under the control of a
    polyhedrin promoter in the vector.

9.  An infected insect cell comprising the recombinant
    vector of claim 7 which synthesizes and actively or passively secretes
    Hepatitis B virus surface antigen comprising DNA
    encoding said surface antigen in frame with the
    polyhedrin promoter and containing transcription
    termination signals, such that said surface
    antigen is synthesized under the control of the
    polyhedrin promoter, said surface antigen being
    recoverable therefrom.

0228036

Eco RI – I

FIG. 1

FIG. 2

FIG. 3

FIG. 4

FIG. 5